# EUROPEAN PATENT APPLICATION

(11) **EP 4 537 898 A1**
(43) Date of publication of application: **16.04.2025**
(21) Application number: 23202467.9
(22) Date of filing: 09.10.2023
(51) Int. Cl.: A61N 5/10

(54) **DOSE MEASUREMENT FOR MONITORING MEDICAL RADIATION DEVICES**

(71) Applicant: Alber, Markus, 72379 Hechingen (DE)
(72) Inventor: Alber, Markus, 72379 Hechingen (DE)
(74) Representative: Paustian & Partner Patentanwälte mbB

(57) **Abstract**

A first aspect of this disclosure is related to a computer-implemented method for monitoring a dose output of a medical radiation device,
comprising the steps:
- obtaining training dose data from a reference device ;
- training a machine learning algorithm, MLA , with the training dose data to teach the MLA to identify the reference device based on dose data from the reference device that differs from the training dose data ;
- obtaining operational dose data from a medical radiation device to be monitored;
- providing the operational dose data to the trained MLA;
- providing output information related to the difference between the reference device and the medical radiation device based on the MLA.

## Description

### Technical Field

The present disclosure is related to methods for monitoring a dose output of a medical radiation device, devices for training a machine learning algorithm for monitoring a dose output of a medical radiation device, devices for monitoring a medical radiation device, and medical radiation devices to be monitored by a method with a machine learning algorithm.

### Background

Medical linear accelerators produce high-energy photon radiation that is used to treat tumor diseases. These devices are individually different. That is, the radiation properties of different devices differ from device to device. To characterize and configure a proper beam for a medical treatment, the radiation properties of a device have to be analyzed.

Radiation properties can be analyzed by dose measurements. These are usually made in a water tank to identify emitted particles (e.g., photons, electrons, positrons) by the release of energy when they collide with other matter. Such dose measurements are inherently noisy. Measurements can also be acquired in solid bodies, with single point detectors or arrays of detectors.

Furthermore, dose measurements are normally perturbed by errors. Failures can be due to measurement errors, e.g., due to the fact that detectors used are not ideal. In addition, configuration errors and operational errors during a measurement procedure can occur. Furthermore, radiation devices are often imperfectly tuned. Therefore, in practice dose measurements can have common and individual errors.

Although there is a large number of guidelines for the measurement of dose data, there are few methods to assure their quality. In particular plausibility checks or standard statistical analyses are often afflicted by errors themselves and are not accurate enough.

Measurement data usually form the input to algorithms or are used to configure algorithms that compute a dose imparted on a patient, e.g., for a cancer therapy. Hence measurement data usually form the input to algorithms that compute the dose imparted on a patient for the cancer therapy. Faulty measurements can lead to a faulty configuration of these algorithms.

Inaccurate dose computation for patient treatments is a problem for linear accelerator-based therapies. Improvement in this field is desirable.

### Summary

An object of this disclosure is to improve a medical radiation therapy.

This object is solved by the disclosed embodiments, which are defined in particular by the subject matter of the independent claims. The dependent claims provide for further embodiments. Various aspects and embodiments of these aspects are also disclosed in the summary and description below, which provide additional features and advantages.

A first aspect of this disclosure is related to a computer-implemented method for monitoring a dose output of a medical radiation device,
comprising the steps:
- obtaining training dose data from a reference device ;
- training a machine learning algorithm, MLA , with the training dose data to teach the MLA to identify the reference device based on dose data from the reference device that differs from the training dose data ;
- obtaining operational dose data from a medical radiation device to be monitored;
- providing the operational dose data to the trained MLA;
- providing output information related to the difference between the reference device and the medical radiation device based on the MLA.

In general, dose data of a medical radiation device stem from a complex quantum-mechanical process whereby high energy particles pass through matter and dissipate their energy in a stochastic fashion. Dose data comprise randomness and/or probability.

Dose data can be provided in terms of an output factor (OF) that provides a dose of a medical radiation device normalized to a calibration dose. An OF can also be termed as "relative dose" or "relative dose factor". For a given (photon) beam at a given source-to-surface distance, a dose output at a certain depth in a medical radiation device normally depends on the field size. An OF can be defined as the ratio of a dose for any field size (e.g. given as length x width in cm^2) to the dose for a reference field at the same source-to-surface distance and at the same depth in a water tank. A reference field can be a square field, e.g. a field of a size 10 ×10 cm^2 at a source-to-surface distance of 100 cm. The OF can be considered as an intrinsic characteristic of the accelerator and can be measured periodically to make sure that the accelerator is operating properly in order to treat patients. Output factors or output quantities with other definitions can also be used.

Dose data can also be provided in terms of a depth-dose-curve (DDC). A DDC provides a dose profile of a medical radiation device along the direction of the beam. For a given (photon) beam at a given source-to-surface distance, a DDC is obtained by scanning the dose along the beam between the surface of the water phantom and a maximum depth determined by the dimensions of the phantom. The DDC also depends on field size much like an OF and can be considered as an intrinsic characteristic of the accelerator and can be measured periodically to make sure that the accelerator is operating properly in order to treat patients. Depth dose curves and/or quantities with other definitions can also be used.

Dose data can also be provided in terms of dose cross profiles (CP). A CP provides a dose profile of a medical radiation device perpendicular to the direction of the beam. For a given (photon) beam at a given source-to-surface distance, CPs are obtained by scanning the dose perpendicular to the beam at various depths, e.g. 5 cm, 10 cm, and 20 cm. The CP also depends on field size much like the DDC and OF and can be considered as an intrinsic characteristic of the accelerator and can be measured periodically to make sure that the accelerator is operating properly in order to treat patients. Cross profiles or quantities with other definitions can also be used.

The radiation beam of a medical radiation device is usually characterized by a multitude of OF, DDC and CPs. These data provide the information to tune the beam properly and configure dose computation algorithms to represent the specific medical radiation device.

Additionally or alternatively, dose data can be provided in terms of absolute units, such as Gray (Gy). Gray (Gy) is a unit of absorbed dose, which quantifies the amount of radiation energy absorbed by a specific material, such as the patient's body tissues. It is a measure of the actual energy deposited per unit mass of the material.

Training dose data can be obtained to have information about a radiation process of a medical radiation device that is not afflicted with failure. The training dose data therefore represents a ground truth against which dose data of the medical devices to be monitored is compared to. Additionally or alternatively, training dose data can be free only of a pre-defined failure, such as a failure of a measurement amplifier. Such a training dose data can provide a ground truth relative to the pre-defined failure. Training dose data from a reference device can comprise the device itself or together with other devices that are important for the analysis. Such a device can be one or more measurement devices.

Training dose data can be obtained for different field sizes (e.g., from a field size of 1cm x 1 cm to a field size from 50 cm x 50 cm). Additionally or alternatively, training dose data can be obtained for different intensities and/or depths, e.g. in a water tank.

In order to capture the aspects of a radiation process of a medical radiation device, a machine learning algorithm, is trained with the training dose data. The learned process is then compared, in terms of its output to an output of a medical radiation device to be monitored. Or, in other words, the machine learning algorithm checks if an output of a medical radiation device could also come from the flawless reference device learned.

Output of a medical radiation device to be monitored is called "operational dose data". Operational dose data can be obtained by measuring the output of a medical radiation device during a real use and/or during a test use. The operational dose data can be obtained for various parameters such as different intensities, different field sizes, different hardware components, e.g. different amplifier, different collimator sizes. In particular, operational data can be used with a certified hardware, to exclude certain hardware failures, such as failures of a beam steering circuits and/or an amplifier attached to the medical dosimetry equipment.

The obtained operational dose data is then provided, in a subsequent step, to a trained machine learning algorithm. The trained machine learning algorithm compares the operational dose date to the ground truth or to the relative ground truth information that was used to train the machine learning algorithm. The comparison can, e.g., result in a value that represents a probability that the medical radiation device to be monitored comprises a fault. This will be explained in more detail in the following.

A method according to the first aspect can effectively identify flawed or at least unnecessarily noisy dose data and/or flawed or unnecessarily noisy medical radiation devices.

An embodiment of the first aspect is related to a method for monitoring a dose output of a medical radiation device,
wherein the training dose data comprises measured dose data from one or more real medical radiation devices.

Obtaining of training dose data from one or more reference devices can comprise obtaining training dose data from a manufacturer. Additionally or alternatively, training dose data can come from one or more individual reference devices. In particular, a reference device has been checked and tested to work without any failures and/or under required environmental conditions and therefore provides flawless dose data. Such dose data excludes failures by the machine, failures by a human operating the machine and/or failures of the measurement detectors and equipment. Error-free training dose data can in particular be used for unsupervised learning.

Additionally or alternatively, training dose data can be obtained by a plurality of devices of the same type. This can make the training dose data more robust against variation within a group of medical radiation devices from a same type. Additionally or alternatively, training dose data can be obtained from a plurality of devices of different types. This can make the training dose data more robust against variations between medical radiation devices of different types. Additionally or alternatively, training dose data can be obtained for different configurations. This can make the training more robust against variations with respect to a configuration (e.g. radiation intensity, radiation field size, attached hardware components) or a setup of a dose measurement equipment of the medical radiation devices.

Training dose data can also comprise data that comes from one or more devices with failures. Additionally or alternatively, training dose data can comprise one or more pre-defined operating errors. Additionally or alternatively, training dose data can comprise one or more pre-defined detector and/or measurement equipment error. Training of a machine algorithm with defective training dose data can be done in form of supervised learning. Thereby, the defective training dose data can be labelled as flawed, in particular as comprising a pre-defined error. Additionally or alternatively, a direct feedback can be provided such that the machine learning knows that an input was error-free and/or defective.

Training data can also comprise data that was acquired with various types of detectors and/or other measurement equipment. Additionally or alternatively, training dose data can be acquired in different measurement bodies. Additionally or alternatively, training dose data can be acquired with different combinations of detectors, equipment and/or measurement bodies. Training of a machine learning algorithm with variably acquired dose data can be done in form of supervised learning. Thereby, the training dose data can be labelled with the type of detector, equipment, or measurement body. Additionally or alternatively, a direct feedback can be provided such that the machine learning algorithm knows that an input was affected by specific traits of type of detector, equipment, or measurement body.

A training of the machine learning algorithm can comprise supervised learning, unsupervised learning and/or reinforcement learning.

Based on training dose data from one or more real devices, a radiation process of a medical radiation device can be learned with adequate accuracy in order to identify errors from a dose measurement equipment and/or operating errors hidden in the dose data.

An embodiment of the first aspect is related to a method for monitoring a dose output of a medical radiation device,
wherein the training dose data comprises simulated dose data from one or more virtual reference devices.

Simulated training dose data can come from a model of a medical radiation device or from a model of a group of medical radiation devices, e.g. a model can be related to a predefined type of medical radiation devices. Error-free simulated training dose data can in particular be used for unsupervised learning of the machine learning algorithm.

Additionally or alternatively, training dose data can be artificially corrupted, in particular with patterns from known failures. For example, a defective measurement amplifier provides an output that decreases with depth. In this case, error-free and defective training data can be labelled as such. As a further example, a detector type may show a sensitivity that depends on measurement depth or the quality of the measured radiation. This can also be simulated.

Based on training dose data from one or more virtual devices (i.e. models), a radiation process of a medical radiation device can be learned with focus on certain parameters and/or on identifying certain failures.

An embodiment of the first aspect is related to a method for monitoring a dose output of a medical radiation device,
comprising the step:
- providing data of one or more real devices, in particular of the device to be monitored, to the virtual reference device, in particular configuration data and/or input data.

Information from real devices can be related to field size, energy intensity, surrounding temperature, humidity, etc. Configuring a model of a medical radiation device with information from real devices can improve model accuracy.

An embodiment of the first aspect is related to a method for monitoring a dose output of a medical radiation device,
wherein operational dose data is obtained for one or more different configurations of one or more medical radiation devices.

Thereby, failures can be identified relative to different configurations. For example, a failure due to the radiation field being too small relative to the detector area (no ideal point measurement) can be potentially identified better based on a configuration with a small radiation field and/or based on a comparison between a medical radiation device configured with a small radiation field and the same medical radiation device configured with a larger radiation field.

An embodiment of the first aspect is related to a method for monitoring a dose output of a medical radiation device,
comprising the steps:
- adapting a configuration of the medical radiation device to be monitored;
- measuring operational dose data by the monitored medical radiation device; and - providing the generated operational data to the trained MLA.

For example, if certain operational dose data from a medical radiation device to be monitored is identified by the machine learning algorithm to be defective, a configuration, e.g. a hardware configuration, can be changed and a second instance of operational dose data can be provided to the machine learning algorithm to check if the dose data is still evaluated as defective in the same way. If this is the case, the changed parameter cannot account for the defectiveness. In this way, a medical radiation device to be monitored can be adapted until it is considered by the machine learning algorithm as being error-free.

In another example, if operational dose data is identified to be error-free, amendments to the configuration of a medical radiation device that provided the operational dose data can be made to check if the medical radiation device is still considered as error-free. Hence, flawlessness of alternative configurations of a medical radiation device can be analyzed.

An embodiment of the first aspect is related to a method for monitoring a dose output of a medical radiation device,
wherein operational dose data is provided to the MLA until a pre-defined quantity threshold of operational dose data is reached and/or a pre-defined accuracy threshold of the output information is reached.

Based on a threshold, a quick result can be provided by a machine learning algorithm with little computational time. Based on this result, a more detailed analysis can be performed. This detailed analysis can be e.g. performed with a different machine learning algorithm. For example, a detailed analysis can be performed to confirm a pre-defined failure, in particular based on a machine learning algorithm that was trained to identify the pre-defined failure.

An embodiment of the first aspect is related to a method for monitoring a dose output of a medical radiation device,
wherein the machine learning algorithm comprises a plurality of different machine learning algorithms that are trained with the training dose data and
wherein the operational dose data is provided to the different machine learning algorithms.

In order to efficiently confirm a plurality of failure hypotheses, operational dose data can be provided to a plurality of machine learning algorithms that were trained to identify different failures. For example, a first neural network can be configured to identify a failure of an amplifier used for the medical radiation device. A second deep learning neural network can be configured to identify a failure related to the field size used relative to the size of the detector. A third neural network can be configured to identify a pre-defined operational error committed by a human operator.

In particular, a use of a plurality of differently configured machine learning algorithms can be based on a prior threshold-based analysis that identified the provided operational dose data as defective. In another embodiment, operational dose data of a medical radiation device can be directly provided to a plurality of differently trained machine learning algorithms to check the dose data for different failures without a prior general analysis. A provision of the operational dose data (or data based on the operational dose data) to the different machine learning algorithms can be performed at least partly concurrently to reduce monitoring time.

An embodiment of the first aspect is related to a method for monitoring a dose output of a medical radiation device,
wherein the MLA comprises a neural network, in particular a fully connected neural network and/or a convolutional neural network.

A neural network used within a machine learning algorithm according to the first aspect can be a neural network with a plurality of hidden layers, also called a deep neural network. In particular a deep neural network can be trained by unsupervised learning. Thereby, the network can learn the features of error-free and/or of defective dose data by itself.

Based on a neural network, in particular a deep neural network, monitoring of a medical radiation device can be performed in an effective and robust manner.

An embodiment of the first aspect is related to a method for monitoring a dose output of a medical radiation device,
wherein the MLA comprises a classificator and/or a regressor.

Once a classifier has learned from the training data, it can be used to classify operational dose data. In particular classification can be performed between the categories 'error-free' and 'defective' dose data / medical radiation device. Additionally or alternatively, classification can be performed in relation to a type of defect in a defective dose data. Additionally or alternatively, a classifier can be used for decision-making related to a processing of an operational dose data. For example, if a dose data is defective, a classifier can make the decision to provide the operational dose data, or at least data based on the operational dose data, to one or more additional machine learning algorithms, in particular one or more machine learning algorithms that are fine-tuned to identify a pre-defined defect of the medical radiation device and/or a pre-defined operational error based on the provided data and/or dose errors introduced by a certain type of detector.

Additionally or alternatively, a regressor can predict a continuous variable, such as a probability or a quantity of a defect identified based on an analyzed operational dose data. In combination with a pre-defined threshold, a regressor can be used for a decision-making process, for example as described above. In one embodiment, a regressor can predict a time-to-maintenance for a monitored medical radiation device.

An embodiment of the first aspect is related to a method for monitoring a dose output of a medical radiation device,
wherein the response from the MLA comprises a classification related to one or more predetermined failure types of the medical device to be monitored.

Based on a classificator, the machine learning algorithm can provide a categorical statement about the operational dose data provided. A categorical statement can be, e.g., that an operational dose data is from an error-free device, or not and/or comprises an operational error, or not.

An embodiment of the first aspect is related to a method for monitoring a dose output of a medical radiation device,
wherein the output information comprises at least one of:
- a probability that the operational dose data is defective and/or is error free;
- a probability that the operational dose data is from the reference device and/or from the medical radiation device to be monitored;
- a time-to-maintenance for the medical radiation device to be monitored.

A probability that the operational dose data is defective and comprise a probability that the operational dose data comprises a pre-defined error, e.g. a pre-defined machine-related error and/or a pre-defined operation-related error. Such an analysis can be provided by a regressor as part of the machine learning algorithm.

An embodiment of the first aspect is related to a method for monitoring a dose output of a medical radiation device, a measurement of operational data from the medical device to be monitored and a provision of the operational data to the MLA are performed at least partly concurrently.

By performing a measurement process and an analysis process at least partly concurrently, a medical radiation device can be monitored during operation and/or under soft or even hard real-time conditions. This allows for detection of errors during a radiation procedure and therefore can protect a patient against failures that occur during a radiation therapy.

A second aspect of this disclosure is related to a computer-implemented method for training a machine learning algorithm for monitoring a dose output of a medical radiation device,
configured to:
- obtain training dose data from a reference device ;
- train a machine learning algorithm, MLA , with the training dose data to teach the MLA to identify the reference device based on dose data from the reference device that differs from the training dose data ;
- provide the trained MLA to a device for monitoring a dose output.

In case the machine learning algorithm comprises several algorithms, e.g. several deep neural networks, a training can be performed related to each of the machine learning algorithms together or separately. In particular, if different machine learning algorithms are related to identifying different failures, then each of the machine learning algorithms can be trained separately. A method for training can comprise all features necessary for providing a trained machine learning algorithm for the method according to the first aspect.

A third aspect of this disclosure is related to a device for monitoring a medical radiation device,
configured to:
- obtain operational dose data from a medical radiation device to be monitored;
- provide the operational dose data to a trained machine learning algorithm, MLA , configured to output information related to a difference between a reference device and the medical radiation device based on the operational dose data ;
- provide the output information for a user.

A device according to the third aspect can comprise one or more functions described in relation to the first aspect. In particular, a machine learning algorithm can comprise a plurality of machine learning algorithms, in particular deep neural networks, wherein a single machine learning algorithm is trained to identify a failure of the medical radiation device in general and/or several machine learning algorithms are trained to identify certain pre-defined errors.

A device according to the third aspect can in particular be a software-implemented device. Additionally or alternatively, a device according to the third aspect can comprise an application user interface (API) to be configured to obtain information and monitor a specific medical radiation device. Additionally or alternatively, the device can be configured to assess an API of a medical radiation device. Thereby, a plurality of medical radiation devices can be monitored.

A fourth aspect of this disclosure is related to a medical radiation device, configured to:
- operate a method according to one of the preceding claims;
- interface with an application user interface of a device operating a method according to one of the preceding claims and/or with an application user interface of a device according to one of the preceding claims.

This aspect is related to the preceding aspects and embodiments can comprise one or more of the aforementioned features.

### Short Description of Figures

Further advantages and features result from the following embodiments, some of which refer to the figures. The figures do not always show the embodiments to scale. The dimensions of the various features may be enlarged or reduced accordingly, in particular for clarity of description. For this purpose the figures are partially schematized:
Fig. 1 illustrates a method for monitoring a medical radiation device.
Fig. 2 illustrates a block diagram of a device for monitoring a medical radiation device.
Fig. 3 illustrates a block diagram of a device for monitoring a medical radiation device.
Fig. 4 illustrates a block diagram of a device for monitoring a medical radiation device.
Fig. 5a and Fig. 5b illustrate diagrams of a monitoring of a medical radiation device.
Fig. 6a and Fig. 6b illustrate diagrams of a monitoring of a medical radiation device.
Fig. 7 illustrates a workflow for a system, for monitoring a medical radiation device.
Fig. 8 illustrates principles of a radiation dose measurement.
Fig. 9 illustrates a medical radiation device with measurement and IT equipment.

In the following description reference is made to the accompanying figures which form part of the disclosure, and which illustrate specific aspects in which the present disclosure can be understood. Identical reference signs refer to identical or at least functionally or structurally similar features.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Detailed Description

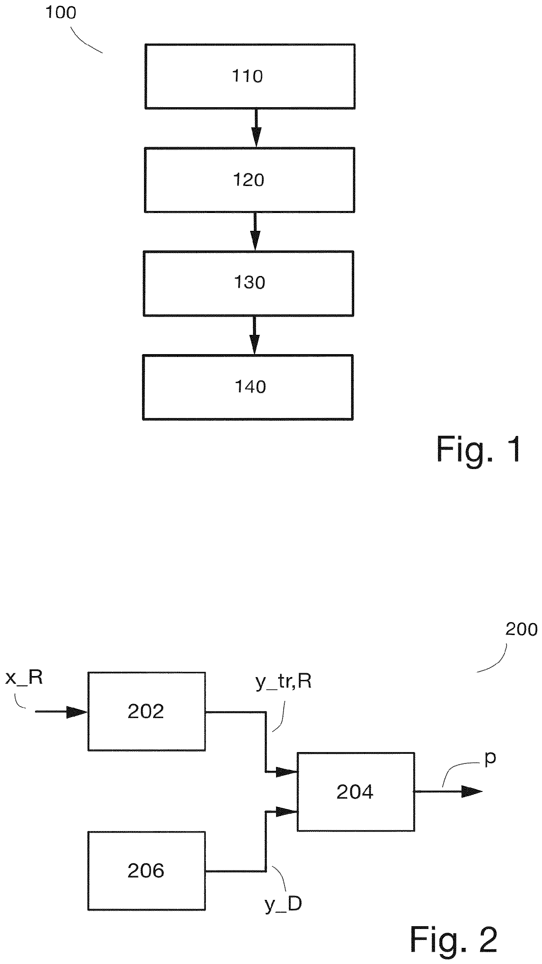

Fig. 1 illustrates method 100 for monitoring a medical radiation device according to an embodiment of the first aspect of this disclosure.

Dose measurements that characterize beam properties of medical linear accelerators are frequently flawed, which can lead to inaccurate dose computation, e.g., for patient cancer treatments. This has been identified as a major cause for sub-standard patient treatments.

Although there is a large number of guidelines for the acquisition of such dose data, there is no method to assure their quality. There are plausibility checks against other data that may also be flawed and if they are not, may be not accurate enough because data variability is too high. Vendors also circulate "golden" standardized beam data that have been showed to be affected by measurement errors, too. Statistical descriptions of measured dose data has been published but are too coarse to allow for effective assessments of operational dose data from a medical radiation device.

In a first step 110 training dose data from one or more real or virtual reference devices is obtained. The training dose data is used to train a machine learning algorithm. Thereby, the machine learning algorithm is taught to identify the reference device based on input dose data that is error free and to distinguish the reference device from devices that are not error-free, i.e. that provide data that is somehow defective (e.g., data is flawed and/or a hardware and/or software of the device is defective). This is done by learning the features, in particular the deterministic and/or non-deterministic features of an error-free medical radiation device based on its ideal error-free dose data. Additionally, the machine learning algorithm can learn deterministic features and/or non-deterministic features of defective medical radiation devices.

In a second step 120 operational dose data from a medical radiation device (106) to be monitored is obtained. The operational dose data is obtained either during a test procedure and/or during operation in a real procedure.

In a third step 130 the obtained operational dose data is provided to the trained machine learning algorithm to analyze if the medical radiation device can be distinguished from the reference device or not. In case, the machine learning algorithm cannot distinguish the medical device to be monitored from the reference device, the operational dose data is considered error-free. Otherwise, it is considered defective.

In a fourth step 140 the result of the analysis is provided as output information for further processing. Alternatively the result can be provided to a user such that a decision can be taken if the medical radiation device can be used or not. Additionally or alternatively, an output can also provide corrected dose data and/or corrected configuration data that can be used as input information for the medical radiation device to be monitored and/or for similar devices. In particular, a dose data correction and/or configuration data correction can be used in a real-time hardware-in-the-loop procedure.

Based on the described method, flawed dose measurements and/or defective medical radiation devices can be identified, their errors can be qualified, quantified, and corrected. In particular, errors of dose measurements can be eliminated if the data is intended to be entered into dose computation software that creates a beam parameters for a patient treatment. Since the method can operate in real time, measurements can be verified while they are taken, making the measurement process more efficient. Additionally, operators can be advised of a likely source of error.

Fig. 2 illustrates a block diagram structure 200 of a device according to the present disclosure. Input data x_R is provided to a reference device 202. The input data x_R can be, e.g., configuration data. The reference device 202 can be a real reference device or a virtual reference device. A virtual reference device can comprise features of real (reference) devices as well. The reference device 202 can also comprise features of a group of real medical radiation devices. The reference device 202 is an error-free reference device. The reference device 202 produces an output y_tr,R. This data is used as training data to train a machine learning algorithm 204, e.g. a neural network, to identify the features of an error-free reference device. The machine learning algorithm can additionally receive training data from defective real and/or virtual reference devices in order to sharpen its ability to differentiate between error-free and defective medical radiation devices. The machine learning algorithm is provided with operational dose data y_D from a medical radiation device 206. This information is analyzed with respect to the learned flawless reference device and/or flawless operational data. The machine learning algorithm provides an output p comprising information that the medical radiation device to be monitored is error-free or defective. Additionally or alternatively, the output can comprise information about a probability that the monitored medical radiation device is defective or error-free.

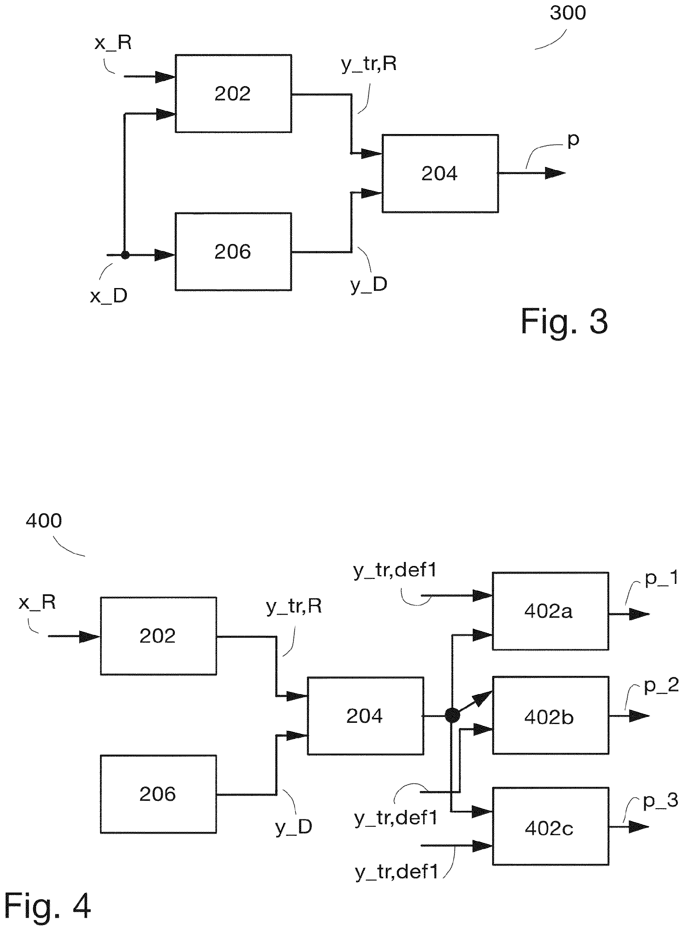

Fig. 3 illustrates a block diagram 300, which is a slightly adapted version of the block diagram 200 of Fig. 2. The system depicted by the block diagram 300 equals the system of the block diagram 200. Additionally, input data x_D of the medical radiation device to be monitored is fed into the reference device 202 as well. In this case, training data y_tr,R can be generated for the machine learning algorithm 204 that comprises information of the real situation of the medical device to be monitored. Such input data x_D can be in particular configuration information (e.g. intensity, field size, hardware components, measurement devices, etc.) or environmental information (e.g. device temperature).

Fig. 4 illustrates a block diagram 400 of another embodiment according to the present disclosure. In this system of the block diagram 400 is based on the system of the block diagram 200 illustrated in Fig. 2. Additionally to the neuronal network 204, the machine learning algorithm comprises three error sensitive neural networks 402a, 402b, 402c. The additional network layer functions as follows. The error-sensitive neural networks 402a, 402b, 402c are each trained with the training data y_tr,def1, y_tr,def2, y_tr,def3 to identify a pre-defined error of a medical device. Therefore, dose data comprising a respective error is used as training data for each network. The defective dose data can be provided from one or more real medical radiation devices and/or by a model, i.e. by a virtual medical device. If the neural network 204 identifies the operational dose data as defective, the operational dose data y_D (or data based on the operational dose data) is provided to the trained error-sensitive neural networks 402a, 402b, 402c concurrently. Each of these neural network then analyzes the operational dose data y_D with respect to the defect it was trained for and provides a categorization p_1, p_2, p_3 whether the operational dose data comprises the respective error or not. Additionally or alternatively p_1, p_2, p_3 can also comprise a probability that the operational dose data y_D comprises a respective error.

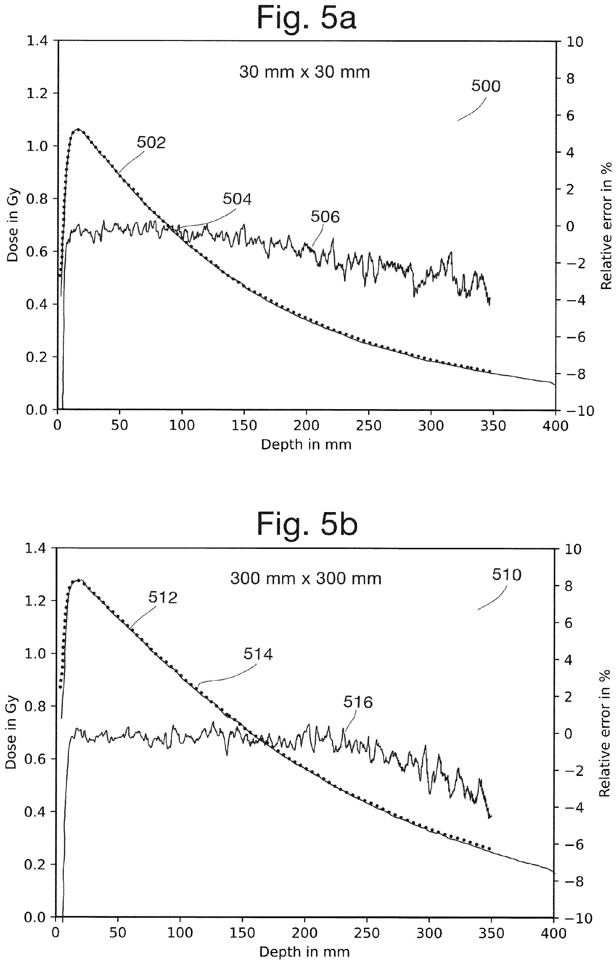

Figs. 5a and 5b shows information generated during a monitoring of a medical radiation device with a method and system according to an embodiment of the present disclosure. In the two diagrams 500, 510, the x-axis represents a radiation penetration depth (ranging from 0 to 400 mm), the left y-axis represents the dose output, and the left y-axis represents a relative error between a virtual output and a measured operational depth dose. The solid line 502, 512 represents a virtual dose output that is generated - only as orientation - with an error-free beam model. The dotted line 504, 514 represents customer measured operational dose data of the medical radiation device to be monitored. The beam model is a generic beam model and is adapted to the output factors measured for the individual medical radiation device. The depth dose curves are provided for a source-surface-distance 950 mm.

In the diagram 500 of Fig. 5a, it can be seen that the predicted dose data and the measured, operational dose data are nearly congruent and differ by a small prediction error 506. The prediction error 516 in diagram 510 of Fig. 5b is similar. In particular, the information comprised in the prediction error is used by the machine learning algorithm (not shown) for the analysis if the provided operational data 502 is error-free or defective. In this case, the failure at larger depths is caused by a malfunctioning measurement amplifier.

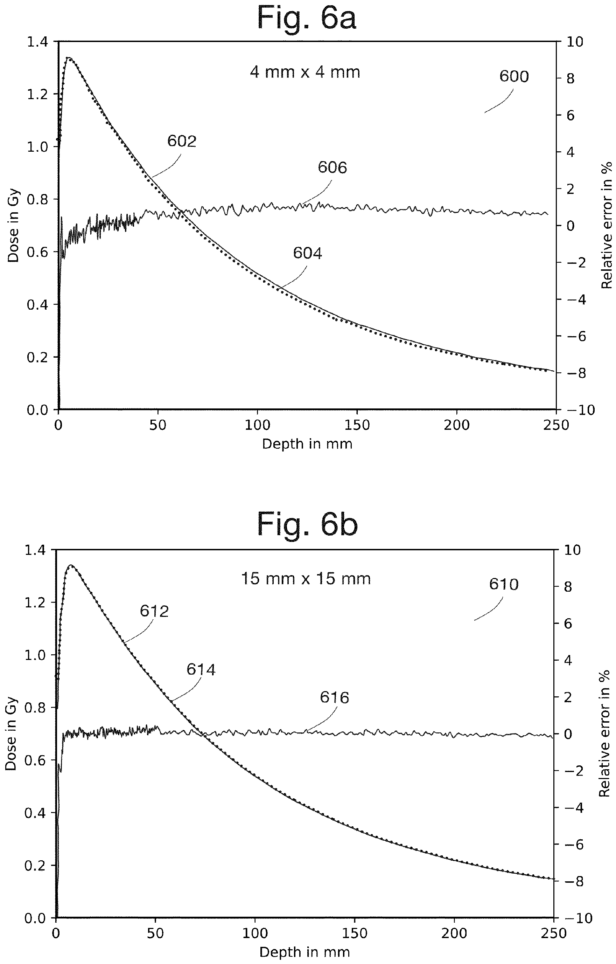

Figs. 6a and 6b show information generated during a monitoring of a medical radiation device with a method and system according to an embodiment of the present disclosure. In the two diagrams 600 and 610, the x-axis represents a radiation penetration depth (ranging from 0 to 250 mm), the left y-axis represents the dose intensity, and the right y-axis represents a relative error between a virtual output and measured operational dose data. The solid line 602, 612 represents the virtual dose output that is generated with an error-free beam model. The dotted line 604, 614 represents customer measurement operational dose data of the medical radiation device to be monitored. The beam model is a generic beam model and is adapted to the output factors measured for the individual medical radiation device. The dose curves are provided for a source-surface-distance 450 mm.

In the diagram 600 of Fig. 6a, it can be seen that the predicted dose data and the measured, operational dose data are nearly congruent and only differ in a small prediction error 606. However, the prediction error 616 in diagram 600 is even smaller. The difference in error is due to the size of the radiation field, which is closer to the size of the detector for the configuration of the medical radiation device in Fig. 6a than for the configuration of Fig. 6b, where the detector is smaller than the field size. By providing both measurements to a trained neuronal network, the neural network can understand that both measurements are defective because of the non-point-like detector size. This error is more serious the smaller the radiation field is, because then the ratio between the size of the detector and the size of the radiation field is closer to 1. Therefore, to facilitate detection of the failure, operational data for different field sizes can be provided to the trained machine learning algorithm and/or for training of the machine learning algorithm.

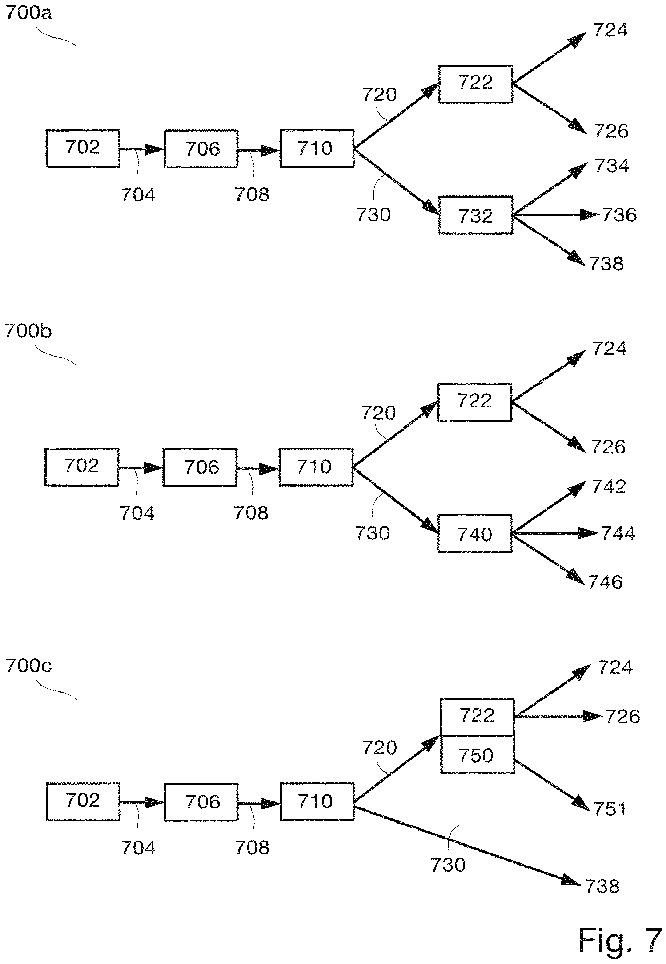

Fig. 7 illustrates workflows of different systems for monitoring a medical radiation device. Alternatively, the workflows can also be realized by a single system. The first workflow/system 700a describes a measurement in a water tank. The linear accelerator 702 emits radiation 704 into a water tank 706 that comprises measurement equipment. The measured data 708 is then provided to a device for monitoring a dose output.

In the device, the information 708 is analyzed by a first machine learning algorithm 710 (MLA1). The MLA1 is trained and configured to analyze if the provided measurement data 708 is 720 flawed or if it is error-free 730. In the first case, if the MLA1 identifies that the measurement data 708 comprises a failure, then the measurement data and/or further data produced by MLA1 is passed to a second machine learning algorithm 722 (MLA2).

MLA2 is trained to identify, based on the obtained data, if the failure within the measurement data results from a failure by an operator 724 or by an equipment 726. In the second case, if MLA1 identifies that the measurement data 708 is failure-free, the measured data and/or further data from MLA1 is passed to a third machine learning algorithm 732 (MLA3).

MLA3 is trained to identify if a beam used for the measurement 706 is tuned correctly 734. Additionally, MLA3 is trained to identify if the beam type used for the measurement 706 needs to be adjusted 736. Additionally MLA3 is trained to identify if the used linear accelerator is faulty 736. For this three-way-analysis, MLA3 can also comprise further differently trained machine learning algorithms. The use of specialized machine learning algorithms can accelerate the generation of the result 724, 726, 734, 736, 738 and increase the rate of correctness for the different result categories. Such an analysis of a medical irradiator can be used to investigate whether the beam characteristics and operator input of a linear accelerator meet specifications, and to suggest remedies for measurement errors and beam tuning deviations.

The second workflow/system 700b is similar to the first system in such that data 708 measured about a linear accelerator 702 is analyzed by a first machine learning algorithm 710 to identify if the data comprises a failure 720 or not 730. If the data comprises a failure 720 a second machine learning algorithm 722 is used to figure out if the failure is due to an operator 724 or due to an equipment 726. However, in case the data does not comprise a failure 730, the data and/or further information by the first machine learning algorithm 710 is provided to a fourth machine learning algorithm 740 (MLA4).

MLA4 is trained and configured to analyze if even though the measurement data is error-free, the linear accelerator 702 used is ok or faulty 742. Furthermore, the MLA4 is trained and configured to analyze if the provided measurement data 708 contains information that a type of preemptive maintenance is due 744. Further, the MLA4 is configured to identify what cause of defect has led to a faulty linear accelerator 746. In sum, by the arrangement 700b it can be verified by measurement whether beam properties of a medical radiation device comply with specifications.

The third workflow/system 700c illustrates a further embodiment that is based on the same measurement workflow 702 - 708 and a first machine learning algorithm 710 that identifies whether a measured data comprises an error 720 or is error-free 730. In the first case 720, the measurement data and/or further information by MLA1 is provided in parallel to two different machine learning algorithms.

The MLA2 is used as in workflows/systems 700a, 700b to identify if the provided data 708, 720 is based on an operator error 724 or an equipment error 726. Additionally, the data 708, 720 is fed into a fifth machine learning algorithm 750 (MLA5). MLA5 is trained and configured to provide corrected data 751, i.e. it synthesizes new data without the identified failure. Thereby, correct input/measurement data can be identified even in case of a failure. In the second case, i.e. if the measured data is error-free 730, the data is approved. Synthesized data may be created by employing a virtual reference device, which may comprise a beam model that is adapted to the specific device or may comprise a generic beam model and configuring the virtual reference device to a multitude of settings, e.g. different field sizes, measurement setups or add-on accessories. Synthesized data can also be produced for different measurement bodies and in arbitrary positions and orientations that may not be accessible experimentally.

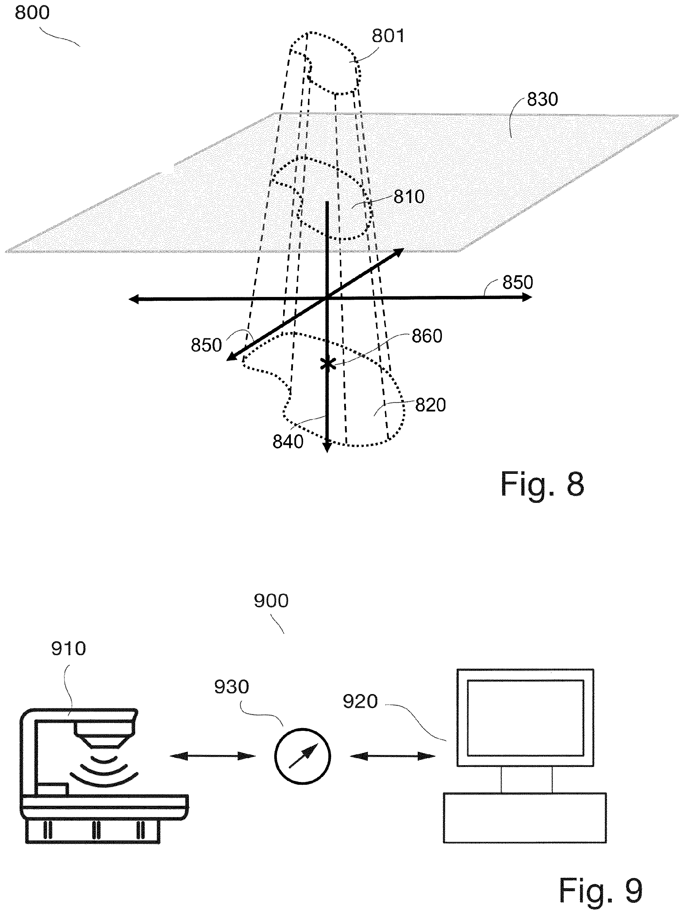

Fig. 8 illustrates principles of a radiation dose measurement 800 in a water tank. After radiation from a radiation source in a treatment machine, an irradiation field 801 has a first form. The form of the irradiation field changes due to radiation dispersion and has a second form 810 when the radiation beams enter water 830 in a water tank. Within the water the radiation beams are further subject to dispersion such that the radiation field has a third form 820 in the plane where the measurement 860 is taken. The coordinate system indicates the three lateral dimensions. The scan direction of the depth dose curve is indicated by dimension 840 and the scan directions of the cross profiles are indicated by dimensions 850.

Fig. 9 illustrates a medical radiation device 910 connected to a system as described in relation to the Figs. 2, 3 or 4. The system 900 comprises a medical radiation device 910 and a computer system 920. The medical radiation device 910 is configured to provide a radiation dose. A measurement device 930 is configured to observe the radiation dose and communicate the measurement information to the computer system 920. The computer system 920 is configured to execute at least a part of a method according to the first aspect of this disclosure. The computer system 920 can be configured to execute a machine learning algorithm and/or to communicate with a machine learning algorithm. The computer 920 and medical radiation device 910 may be separate entities but can also be integrated together in one common housing. The computer 920 may be part of a central processing system of the medical radiation device 910 and/or the computer 920 may be part of a subcomponent of the medical radiation device 910.

The computer system 920 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 920 may comprise any circuit or combination of circuits. In one embodiment, the computer system 920 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 920 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 920 may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system 920 may also include a display device, one or more speakers, and a keyboard and/or controller, which can include a mouse, trackball, touch screen, voice-recognition device, or any other device that permits a system user to input information into and receive information from the computer system 920.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the disclosure can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the disclosure comprise a data carrier having electronically readable control signals, which are capable of cooperating with a programmable computer system, such that one of the methods described herein is performed.

Generally, embodiments of the present disclosure can be implemented as a computer program product with a program code, the program code being operative for performing one of the methods when the computer program product runs on a computer. The program code may, for example, be stored on a machine-readable carrier. Other embodiments comprise the computer program for performing one of the methods described herein, stored on a machine-readable carrier. In other words, an embodiment of the present disclosure is, therefore, a computer program having a program code for performing one of the methods described herein, when the computer program runs on a computer.

A further embodiment of the present disclosure is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present disclosure is an apparatus as described herein comprising a processor and the storage medium.

A further embodiment of the disclosure is, therefore, a data stream or a sequence of signals representing the computer program for performing one of the methods described herein. The data stream or the sequence of signals may, for example, be configured to be transferred via a data communication connection, for example, via the internet.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate, or apply the knowledge.

Machine-learning algorithms are usually based on a machine-learning model. In other words, the term "machine-learning algorithm" may denote a set of instructions that may be used to create, train, or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Signs

- 100: method monitoring medical radiation device
- 110: first step
- 120: second step
- 130: third step
- 140: fourth step
- 200: block diagram
- 202: reference device
- 204: neural network
- 206: medical radiation device
- 300: block diagram
- 400: block diagram
- 402a: error-sensitive neuronal-network
- 402b: error-sensitive neuronal-network
- 402c: error-sensitive neuronal-network
- 500: verification diagram
- 502: virtual dose output
- 504: operational dose data
- 506: prediction error
- 510: verification diagram
- 512: virtual dose output
- 514: operational dose data
- 516: prediction error
- 600: diagram
- 602: error free virtual dose output
- 604: operational dose data
- 606: prediction error
- 610: diagram
- 612: error free virtual dose output
- 614: operational dose data
- 616: prediction error
- 700a: first workflow or system
- 700b: second workflow or system
- 700c: third workflow or system
- 702: linear accelerator
- 704: irradiation
- 706: water tank
- 708: measurement data
- 710: MLA1
- 720: flawed measurement data
- 722: MLA2
- 724: operator error
- 726: equipment error
- 730: failure free measurement data
- 732: MLA3
- 734: beam tune correct
- 736: linear accelerator faulty
- 740: MLA4
- 742: linear accelerator ok
- 744: type of preemptive maintenance
- 746: cause of defect of linear accelerator
- 750: MLA5
- 751: approved measurement data
- 800: radiation dose measurement
- 801: irradiation field first form
- 810: irradiation field second form
- 820: irradiation field in water
- 830: water surface
- 840: scan direction of depth dose curve
- 850: scan direction of cross profiles
- 860: point of measurement of output factor
- 900: medical radiation system
- 910: medical radiation device
- 920: computer
- 930: measurement device

## Claims

1. Method for monitoring a dose output of a medical radiation device, comprising the steps:
- obtaining training dose data (y_tr,R) from a reference device (202);
- training a machine learning algorithm, MLA (104), with the training dose data (y_tr,R) to teach the MLA to identify the reference device based on dose data from the reference device (y_R) that differs from the training dose data (y_tr,R);
- obtaining operational dose data (y_D) from a medical radiation device (106) to be monitored;
- providing the operational dose data (y_D) to the trained MLA;
- providing output information (p) related to the difference between the reference device and the medical radiation device based on the MLA.

2. The method according to the preceding claim,
wherein the training dose data (y_tr,R) comprises measured dose data (y_R1, y_R2) from one or more real medical radiation devices (106).

3. The method according to one of the preceding claims,
wherein the training dose data (y_tr,R) comprises simulated dose data (y_tr,Rv) from one or more virtual reference devices (102).

4. The method according to the preceding claims,
comprising the step:
- providing data of one or more real devices, in particular of the device to be monitored, to the virtual reference device, in particular configuration data (p_R1, p_R2, P_D) and/or input data (x_R1, y_R2, X_D).

5. The method according to one of the preceding claims,
wherein operational dose data (y_d) is obtained for one or more different configurations of one or more medical radiation devices (106).

6. The method according to one of the preceding claims,
comprising the steps:
- adapting a configuration of the medical radiation device (106) to be monitored;
- measuring operational dose data (y_D,2) by the monitored medical radiation device; and
- providing the generated operational data (y_D,2) to the trained MLA (104).

7. The method according to one of the preceding claims,
wherein operational dose data (y_d) is provided to the MLA (104) until a pre-defined quantity threshold of operational dose data is reached and/or a pre-defined accuracy threshold (a) of the output information (p) is reached.

8. The method according to one of the preceding claims,
wherein the machine learning algorithm comprises a plurality of different machine learning algorithms that are trained with the training dose data (y_tr,R) and wherein the operational dose data (y_d) is provided to the different machine learning algorithms.

9. The method according to one of the preceding claims,
wherein the MLA (104) comprises a neural network, in particular a fully connected neural network and/or a convolutional neural network.

10. The method according to one of the preceding claims,
wherein the MLA (104) comprises a classificator and/or a regressor.

11. The method according to one of the preceding claims,
wherein the response from the MLA (104) comprises a classification related to one or more predetermined failure types of the medical device to be monitored.

12. The method according to one of the preceding claims,
wherein the output information comprises at least one of:
- a probability (p) that the operational dose data (y_d) is defective and/or is error free;
- a probability (p) that the operational dose data (y_d) is from the reference device (102) and/or from the medical radiation device to be monitored;
- a time-to-maintenance for the medical radiation device to be monitored.

13. The method according to one of the preceding claims,
a measurement of operational data (y_d) from the medical device to be monitored and a provision of the operational data to the MLA (104) are performed at least partly concurrently.

14. A device for training a machine learning algorithm, MLA, for monitoring a dose output of a medical radiation device,
configured to:
- obtain training dose data (y_tr,R) from a reference device (102);
- train a machine learning algorithm, MLA (104), with the training dose data (y_tr,R) to teach the MLA to identify the reference device based on dose data from the reference device (y_R) that differs from the training dose data (y_tr,R);
- provide the trained MLA to a device for monitoring a dose output.

15. A device for monitoring a medical radiation device,
configured to:
- obtain operational dose data (y_D) from a medical radiation device (104) to be monitored;
- provide the operational dose data (y_D) to a trained machine learning algorithm, MLA (104), configured to output information (p) related to a difference between a reference device (102) and the medical radiation device (104) based on the operational dose data (y_D);
- provide the output information (p) for a user.

16. A medical radiation device,
configured to:
- operate a method according to one of the preceding claims;
- interface with an application user interface of a device operating a method according to one of the preceding claims and/or with an application user interface of a device according to one of the preceding claims.
